(19) Europäisches Patentamt
European Patent Office
Office européen des brevets

(11) Publication number: 0 355 213 A1

# (12) EUROPEAN PATENT APPLICATION

(21) Application number: 88306610.2

(22) Date of filing: 17.08.88

(51) Int. Cl.4: B01J 29/00 , //C07C2/12, C07C15/00

(43) Date of publication of application: 28.02.90 Bulletin 90/09

(84) Designated Contracting States: BE DE FR GB IT NL

(71) Applicant: MOBIL OIL CORPORATION
150 East 42nd Street
New York New York 10017(US)

(72) Inventor: Aufdembrink, Brent Allen
10 Madleyn Avenue
Wilmington, Delaware 19803(US)
Inventor: Degnan, Thomas Francis, Jr.
40 North Homestead Drive
Yardley Pennsylvania 19067(US)
Inventor: McCullen, Sharon Brawner
119 Colonial Drive
Newton Pennsylvania 18940(US)

(74) Representative: Colmer, Stephen Gary
Patent Department c/o Mobil Services Company Limited Mobil Court 3 Clements Inn
London WC2A 2EB(GB)

## (54) A catalyst comprising a porous crystalline silicate and a pillared layered metal oxide and its use in the production of aromatic hydrocarbons.

(57) A catalyst composition suitable for use in aromatization of non-aromatic hydrocarbons comprises a shape-selective crystalline silicate such as ZSM-5 and a titanometallate-type layered metal oxide comprising a layered metal oxide and pillars of an oxide of at least one element selected from Groups IB, IIB, IIIA, IIIB, IVA, IVB, VA, VB, VIA, VIIA and VIIIA of the Periodic Table of the Elements separating the layers of the metal oxide where each layer of the metal oxide has the general formula

$[M_x\square_y Z_{2-(x+y)}O_4]^{q-}$

wherein M is at least one metal of valence n wherein n is an integer between 0 and 7, □ represents a vacancy site, Z is a tetravalent metal, and wherein

$q = 4y-x(n-4)$

$0< x+y <2$

EP 0 355 213 A1

# A CATALYST COMPRISING A POROUS CRYSTALLINE SILICATE AND A PILLARED LAYERED METAL OXIDE AND ITS USE IN THE PRODUCTION OF AROMATIC HYDROCARBONS

The present invention relates to a catalyst comprising a porous crystalline silicate and a pillared layered metal oxide and its use in the production of aromatic hydrocarbons.

The production of aromatic hydrocarbons from non-aromatic hydrocarbons using shape-selective catalyst materials is well-known. U.S. Patent No. 3,756,942 teaches such a method using a zinc-exchanged ZSM-5, whereas U.S. Patent No. 4,180,689 teaches conversion of $C_3$-$C_{12}$ hydrocarbons to aromatics using a zeolite, such as ZSM-5, ZSM-11, ZSM-12 or ZSM-35 which is gallium-exchanged or impregnated. Although the incorporation of a metal of mild dehydrogenation function such as zinc or gallium activates the catalyst for aromatization reactions, loss of the metal, for example by elution, commonly occurs under the high temperature reducing conditions encountered in aromatization. U.S. Patent No. 4,490,569 teaches a method to reduce such elution by incorporating gallium as well as zinc into a zeolite aromatization catalyst.

It has now been found that loss of the dehydrogenation metal in these aromatization catalysts can be limited by utilizing a composite aromatization catalyst which comprises a shape-selective crystalline silicate and a layered metal oxide of the titanometallate-type, such as titanozincate or titanogallate, containing between its layers a polymeric chalcogenide such as polymeric silica. Since the dehydrogenating metal is present in the framework of the titanometallate-type layered metal oxide, it is relatively resistant to loss by elution or other means. Moreover, the reducing metal in the titanometallate-type layered metal oxide framework is inherently well-dispersed throughout the high surface area material.

The placing of dehydrogenation metal functions on non-zeolite supports such as alumina has generally been avoided in the past for various reasons. Undesirable reactions of the metal with the support can often occur, for example, zinc's formation of a catalytically inactive "spinel" structure with alumina. Moreover, achieving adequate dispersion of the metals on such materials, particularly materials which lack ion exchange capacity, is difficult. Finally, excessive migration of reducing metals which are of relatively low melting point occurs when such metals are associated with amorphous or highly siliceous supports. Accordingly, the discovery of a suitable support for the reducing metal which can be physically combined with a shape-selective crystalline silicate represents a significant advance in the field of aromatization catalysts.

Accordingly, the present invention resides in one aspect in a catalyst composition which comprises a) a porous crystalline silicate material, a constraint index of at least 1, preferably 1 to 12, and b) a pillared metal oxide material comprising a layered metal oxide and pillars of a chalcogenide of at least one element selected from Groups IB, IIB, IIIA, IIIB, IVA, IVB, VA, VB, VIA, VIIA and VIIIA of the Periodic Table of the Elements (Fisher Scientific Co. Cat. No. 5-702-10, 1978) separating the layers of the metal oxide, wherein each layer of the metal oxide has the general formula

$[M_x\square_y Z_{2-(x+y)}O_4]^{q-}$

wherein M is at least one metal of valence n wherein n is an integer between 0 and 7 and preferably is 2 or 3, □ represents a vacancy site, Z is a tetravalent metal, preferably titanium, and wherein

$q = 4y-x(n-4)$ and preferably is 0.6-0.9,

$0<x+y<2$

The pillared metal oxide material normally contains 0.5 to 20 weight percent of said element M, preferably 1 to 10 weight percent.

In a further aspect, the invention resides in a process for producing aromatic hydrocarbons which comprises contacting a feed containing non-aromatic $C_2$ to $C_{12}$ hydrocarbons with the composition of any preceding claim at a pressure of 100-7000 kPa (atmospheric to 1000 psig), a weight hourly space velocity of about 0.05 to 300 and a temperature of about 204 to 675 °C.

The Figure is a graph depicting loss of zinc from various zinc-containing aromatization catalysts, including one of the present invention, over time. Other catalysts set out therein are zinc-impregnated $TiO_2$ and zinc-exchanged ZSM-5.

For purposes of the present invention the term "chalcogenide" includes members of the group consisting of oxides, sulfides, selenides, tellurides, and polonides of elements other than those of Group VIB of the Periodic Table of the Elements (Fisher Scientific Co. Cat. No. 5-702-10, 1978), with oxides being particularly preferred. The term "polymeric chalcogenides" includes chalcogenides of two or more repeating units, preferably three or more repeating units, say four or more or even five or more repeating units. The extent of polymerization of the interspathic polymeric chalcogenide is believed to affect the ultimate interlayer separation of the pillared metal oxide material.

The catalyst composition of the present invention generally contains 5 to 95, preferably 15 to 40, weight

percent of said intercalated titanometallate-type layered metal oxide and 5 to 95, preferably 60 to 85, weight percent of a porous crystalline silicate material having a Constraint Index of at least 1, preferably 1-12. Constraint Index is described in detail in U.S. Patent No. 4016218.

Constraint Index (CI) values for some typical materials are:

| | CI (at test temperature) |
|---|---|
| ZSM-4 | 0.5 (316 °C) |
| ZSM-5 | 6-8.3 (371 °C - 316 °C) |
| ZSM-11 | 5-8.7 (371 °C -316 °C) |
| ZSM-12 | 2.3 (316 °C) |
| ZSM-20 | 0.5 (371 °C) |
| ZSM-22 | 7.3 (427 °C) |
| ZSM-23 | 9.1 (427 °C) |
| ZSM-34 | 50 (371 °C) |
| ZSM-35 | 4.5 (454 °C) |
| ZSM-48 | 3.5 (538 °C) |
| ZSM-50 | 2.1 (427 °C) |
| TMA Offretite | 3.7 (316 °C) |
| TEA Mordenite | 0.4 (316 °C) |
| Clinoptilolite | 3.4 (510 °C) |
| Mordenite | 0.5 (316 °C) |
| REY | 0.4 (316 °C) |
| Amorphous Silica-alumina | 0.6 (538 °C) |
| Dealuminized Y | 0.5 (510 °C) |
| Erionite | 38 (316 °C) |
| Zeolite Beta | 0.6-2.0 (316 °C-399 °C) |

The crystalline silicate material employed in the present invention is preferably selected from the group consisting of ZSM-5 (US Patent No. 3702886), ZSM-11 (US Patent No. 3709979), ZSM-12 (US Patent No. 3832449), ZSM-22 (Canadian Patent No. 1202941), ZSM-23 (US Patent No. 4076842), ZSM-35 (US Patent No. 4016245), ZSM-48 (US Patent No. 4375573), ZSM-50 (US Patent No. 4640849), ZSM-57 (EP-A-212795), ZSM-58 (EP-A-193282), with ZSM-5 being particularly preferred.

The pillared metal oxide material employed in the catalyst composite of the present invention comprises a layered metal oxide and pillars of a chalcogenide, preferably an oxide, of at least one element selected from Groups IB, IIB, IIIA, IIIB, IVA, IVB, VA, VB, VIA, VIIA and VIIIA of the Periodic Table of the Elements (Fisher Scientific Co. Cat. No. 5-702-10, 1978) separating the layers of the metal oxide, wherein each layer of the metal oxide has the general formula:

$[M_x\square_y Z_{2-(x+y)}O_4]^{q-}$

wherein M is at least one metal of valence n wherein n is an integer between 0 and 7 and preferably is 2 or 3, □ represents a vacancy site, Z is a tetravalent metal, preferably titanium, and wherein

$q = 4y-x(n-4)$ and preferably is 0.6-0.9,

$0 < x+y < 2$

The preferred pillared material, wherein the chalcogenide pillars are formed of an oxide, is described in International Publication No. WO 88/00090.

In a particularly preferred embodiment, wherein Z is titanium and there are no vacancy sites, the layered oxide starting material is titanometallate having the general formula $A_x(M_{x/n}Ti_{2-x/n})O_4$, where A is a monovalent cation and n = 1 or 2 provided that : where n=1, 0 is less than x is less than 2 and M is a trivalent cation; where n = 2, 0 is less than x is less than 4 and M is a divalent cation.

Most preferably, the layered titanometallate is a titanogallate or a titanogincate and the fillers are formed of polymeric silica.

The layered product described in the preceding paragraph can be prepared by a method which comprises the steps of starting with said layered metal oxide and physically separating the layers thereof by introducing an organic cationic species between the layers at interlayer anionic sites associated with the layered oxide, introducing between the separated layers of the layered oxide a compound capable of conversion to an oxide and then converting said compound to the oxide to form oxide pillars separating adjacent layers of the layered oxide.

It is to be appreciated that the term "layered" metal oxide is used herein in its commonly accepted sense to refer to a material which comprises a plurality of separate metal oxide layers which are capable of being physically displaced away from one another such that the spacing between adjacent layers is increased. Such displacement can be measured by X-ray diffraction techniques and/or by density measurements.

The pillared titanometallate-type layered metal oxide products can have a relatively high interplanar distance (d-spacing), e.g., greater than about 10Angstrom and preferably greater than 20 Angstrom up to and even exceeding 30Angstrom. These materials are capable of being exposed to severe conditions such as those encountered in calcining, e.g., at temperatures of about 450° C for about two or more hours, e.g., four hours, in nitrogen or air, without significant decrease, say, e.g., less than about 10%, in interlayer distance. Furthermore, such pillared oxides can be prepared without the severe dilution often necessary to introduce the interspathic material in prior art techniques of interlayering. Finally, the size of interspathic oxide contained within the final product can be greatly varied because the oxide precursor species is introduced in an electrically neutral form such that the amount of interspathic material incorporated within the titanometallate-type layered metal oxide is not dependent upon the charge density of the original layered oxide. Charge density should be taken into consideration in determining the suitability of the cationic species introduced between the layers in the procedure used to prop open the layers prior to pillaring.

Interposed between the layers of the metal oxide starting material are charge-balancing cations A of charge m wherein m is an integer between 1 and 3, preferably 1. Preferably A is a large alkali metal cation selected from the group consisting of Cs, Rb and K and M is a divalent or trivalent metal cation selected from at least one Mg, Sc, Mn, Fe, Cr, Ni, Cu, Zn, In, Ga and Al. For example, M can be both In and Ga. Structurally, these metal oxides are believed to consist of layers of $(M_x\square_yZ_{1-x-y})O_6$ octahedra which are trans edge-shared in one dimension and cis edge-shared in the second dimension forming double octahedral layers which are separated by cations in the third dimension. These materials can be prepared by high temperature fusion of a mixture of 1) metal (M) oxide, 2) alkali metal carbonate or nitrate and 3) tetravalent metal (Z) dioxide, e.g., titanium dioxide or by fusion of a mixture of alkali metallate and tetravalent metal dioxide. Such fusion can be carried out in air in ceramic crucibles at temperatures ranging between 600 to 1100° C after the reagents have been ground to an homogeneous mixture. The resulting product is ground to 20 to 250 mesh, preferably about 100 mesh, prior to the organic swelling and polymeric oxide intercalation steps.

Further description of layered titanometallate starting materials and their methods of preparation can be found in the following references:

Reid, A.F.; Mumme, W.G.; Wadsley, A.D. Acta Cryst. (1968), B24, 1228; Groult, D.; Mercy, C.; Raveau, B. J. Solid State Chem. 1980, 32 289; England, W.A.; Burkett, J.E.; Goodenough, J.B.; Wiseman, P. J. J. Solid State Chem. 1983, 49 300.

The titanometallate-type layered metal oxide starting material is initially treated with a "propping" agent comprising a source of organic cation, such as organoammonium cation, in order to effect an exchange of the interspathic cations resulting in the layers of the starting material being propped apart. Suitable organoammonium cations include n-dodecylammonium, n-octylammonium, n-heptylammonium, n-hexylammonium and n-propylammonium. During this propping or swelling step it is important to maintain a low hydrogen ion concentration to prevent decomposition of the titanometallate-type structure as well as to prevent preferential sorption of hydrogen ion over the propping agent. A pH range of 6 to 10, preferably 7 to 8.5 is generally employed during treatment with the propping agent. After this treatment, it has been found advantageous to wash out excess propping agent using a propping agent-soluble reagent followed by washing with water. For example, ethanol is soluble in and hence suitable for use with an n-octylamine propping agent. Such washing permits greater incorporation of the oxide pillar precursor in the layered metal oxide. The water treatment allows penetration of water into the interlayer spaces which assists in subsequent hydrolysis the oxide pillar precursor.

After the ion exchange, the organic-"propped" species is treated with a compound capable of conversion, preferably by hydrolysis, to pillars of an oxide, preferably to a polymeric oxide. Where the treatment involves hydrolysis, this may be carried out using the water already present in organic-"propped" material. In this case, the extent of hydrolysis may be modified by varying the extent to which the organic-"propped" species is dried prior to addition of the polymeric oxide precursor.

It is preferred that the organic cation deposited between the layers be capable of being removed from the pillared material without substantial disturbance or removal of the interspathic polymeric oxide. For example, organic cations such as n-octylammonium may be removed by exposure to elevated temperatures, e.g., calcination, in nitrogen or air, or by chemical oxidation preferably after the interspathic polymeric oxide precursor has been converted to the polymeric oxide pillars in order to form the titanometallate-type layered metal oxide product employed in the present invention.

The products of the present invention, especially when calcined, exhibit high surface area, e.g., greater than 200, 300, 400 or even 600 $m^2/g$, and thermal and hydrothermal stability making them highly useful as catalysts or catalytic supports, for hydrocarbon conversion processes for example, cracking and hydrocracking.

The titanometallate-type layered metal oxide starting material is initially subjected to a swelling or propping step in which the material is treated with an organic compound capable of forming cationic species such as organophosphonium or organoammonium ion, between the oxide layers. Insertion of the organic cation between the adjoining layers serves to physically separate the layers in such a way as to make the layered material receptive to the interlayer addition of an electrically neutral, hydrolyzable, polymeric oxide precursor. In particular, alkylammonium cations have been found useful in the present invention. Thus $C_3$ and larger alkylammonium, e.g., n-octylammonium, cations are readily incorporated within the interlayer spaces of the layered metal oxide serving to prop open the layers in such a way as to allow incorporation of the polymeric oxide precursor. The extent of the interlayer spacing can be controlled by the size of the organoammonium ion employed so that use of the n-propylammonium cation can achieve an interlayer spacing of 2 to 5Angstrom whereas to achieve an interlayer spacing of 10 to 20Angstrom an n-octylammonium cation or a cation of equivalent length is required. Indeed, the size and shape of the organic cation can affect whether or not it can be incorporated within the layered structure at all. For example, bulky cations such as tetrapropylammonium are generally undesirable for use in the present method while n-alkyl ammonium cations such as those derived from n-alkyl primary amines and $R_3R'N^+$ cations where R is methyl or ethyl and R is an n-alkyl group with at least 5 carbon atoms, are preferred. Preferably treatment with the organic cationic species is conducted in aqueous media so that water is then available to hydrolyze the electrically neutral, hydrolyzable polymeric chalcogenide precursor subsequently introduced into the "propped" product.

Interspathic chalcogenide pillars are then formed between the layers of the propped or swollen layered metal oxide starting material and may include a chalcogenide, preferably a polymeric chalcogenide, of zirconium or titanium or more preferably of an element selected from Group IVB of the Periodic Table (Fischer Scientific Company Cat. No. 5-702-10, 1978), other than carbon, i.e., silicon, germanium, tin and lead. Other suitable chalcogenides include those of Group VA, e.g., V, Nb, and Ta, those of Group IIA, e.g., Mg or those of Group IIIB, e.g., B. Most preferably, the pillars include polymeric silica. In addition, the chalcogenide pillars may include an element which provides catalytically active acid sites in the pillars, preferably aluminum.

The chalcogenide pillars are formed from a precursor material which is preferably introduced between the layers of the organic "propped" species as a cationic, or more preferably, electrically neutral, hydrolyzable compound of the desired elements, e.g., those of group IVB. The precursor material is preferably an organometallic compound which is a liquid under ambient conditions. In particular, hydrolyzable compounds, e.g., alkoxides, of the desired elements of the pillars are utilized as the precursors. Suitable polymeric silica precursor materials include tetraalkylsilicates, e.g., tetrapropylorthosilicate, tetramethylorthosilicate and, most preferably, tetraethylorthosilicate. Where the pillars are also required to include a different polymeric metal oxide, e.g., alumina or titania, a hydrolyzable compound of said metal can be contacted with the organic "propped" species before, after or simultaneously with the contacting of the propped titanometallate with the silicon compound. Preferably, the hydrolyzable aluminum compound employed is an aluminum alkoxide, e.g., aluminum isopropoxide. If the pillars are to include titania, a hydrolyzable titanium compound such as titanium alkoxide, e.g., titanium isopropoxide, may be used. In addition, the chalcogenide precursor may contain zeolite precursors such that exposure to conversion conditions results in the formation of interspathic zeolite material as at least part of the chalcogenide pillars. Pillars of polymeric silica and polymeric alumina or polymeric silica and polymeric titania are particularly preferred.

After hydrolysis to produce the chalcogenide pillars and calcination to remove the organic propping agent, the final pillared product may contain residual exchangeable cations. Such residual cations in the layered material can be ion exchanged by known methods with other cationic species to provide or alter the catalytic activity of the pillared product. Suitable replacement cations include cesium, cerium, cobalt, nickel, copper, zinc, manganese, platinum, lanthanum, aluminum, ammonium, hydronium and mixtures thereof.

The resulting pillared products exhibit thermal stability at temperatures of 500 °C or even higher as well as substantial sorption capacities (as much as 10 to 25 wt% for $H_2O$ and $C_6$ hydrocarbon). Silica-pillared products possess interlayer separations of greater than 12A and surface areas greater that 250 $m^2/g$ when

divalent metal atoms, e.g., Mg, Ni, Cu and Zn, are present as the metal M of the product. Silica-pillared products incorporating trivalent metal atoms, e.g., Sc, Mn, Fe, Cr, In, Ga and Al can possess interlayer separations of 6 to 15A. The calcined products of the present invention, particularly those containing interspathic polymeric chalcogenides as prepared by the method of the present invention are suited to use as catalysts for petroleum processing owing to their high surface areas, large interlayer openings, thermal stability and the wide variety of metal atoms which may be incorporated therein.

The invention will now be more particularly described with reference to the following examples.

Example 1 (Comparative)

A HZSM-5 catalyst was prepared by twice exchanging a conventionally prepared NaZSM-5 material having a silica to alumina molar ratio of about 70 with a 1 N $NH_4NO_3$ solution followed by calcining in air at 540° C (1000° F). The calcined catalyst was then streamed at 540° C (1000° F) with n-hexane at a WHSV of 1.4. The composition of the product from this reaction is summarized in Table 1.

Example 2 (Comparative)

A 1% Zn containing ZSM-5 catalyst was prepared from a fresh sample of the same calcined HZSM-5 described in Example 1 by means of impregnation with 0.4 g of $Zn(NO_3)_2 \cdot 6H_2O$ in sufficient deionized water to wet the sample. The sample was dried at room temperature and air calcined at 540° C (1000° F) for two hours. This catalyst was then streamed with n-hexane at 540° C (1000° F) at a WHSV of 1.4. The composition of the products from this reaction is also shown in Table 1.

Example 3 (Comparative)

An 8% Zn containing ZSM-5 catalyst was prepared in an identical manner as described in Example 2 from a fresh sample of the calcined ZSM-5 described in Example 1. This catalyst was then streamed with n-hexane at 540° C (1000° F) at a WHSV of 1.4. The composition of the products from this reaction are also shown in Table 1.

Example 4

HZSM-5 having a silica to alumina molar ratio of about 70 and acidity of 360 as determined by the alpha test was physically composited in a mortar and pestle with a silica-pillared titanozincate material having the properties shown in Table 2. The weight ratio of zeolite to titanozincate was about 3:1 and the resulting mixture contained about 2.35 weight percent zinc. This material was pelleted and sized to 20/80 mesh and was streamed with n-hexane at 540° C (1000° F) at a WHSV of 1.4 on zeolite. Analysis of the products gave the composition shown in Table 1.

The silica-pillared titanozincate material was prepared as follows:

$K_2CO_3$, ZnO and $TiO_2$ were thoroughly ground to form a homogeneous mixture having the stoichiometric $K_{0.80}(Zn_{0.40}Ti_{1.60})O_4$ which was then fired in air in a ceramic crucible at 900° C for 200 minutes and then at 1050° C for 720 minutes. The product was then reground and refired under the same conditions. The resulting stiff powder was then ground to about 100 mesh to yield a product analysed as $K_{0.66}(Zn_{0.35}Ti_{1.49})O_4$. The d-layer spacing from X-ray powder diffraction d(A) was measured as 7.83. Excess octylamine (5 mole equiv/mole equiv of titanozincate) was slowly added to a solution of 12% HCl (4.9 equiv HCl/mole) while keeping the temperature of the reaction mixture below 50° C to form an acidic aqueous octylammonium chloride solution. The titanozincate was then added to the chloride solution and the mixture was heated to reflux for 24 hours. The reaction mixture was cooled, filtered and washed with hot distilled water. The air dried product was a material having the composition

$H_3O^+_{0.33}K_{0.03}(NH_3R^+)_{0.56}[Zn_{0.46}Ti_{1.75}]O_4$

containing 2.68 weight percent N and having a 24.6 A d-layer spacing from the lowest two theta peak in its X-ray diffraction pattern. The formula was calculated from analytical data obtained for the materials, assuming that the $H_3O^+$ content of the interlayer can be derived by subtraction of the total cation content (alkali metal plus octylammonium) from that required to balance the charge on the $(M,Ti)_2O_4^{\,n-}$ layers due

to incorporation of the transition metal cation. A low $H^+$ concentration was maintained during octylammonium exchange to avoid decomposition of the metallotitanate structure.

The octylammonium-exchanged product was then stirred in EtOH for 2 hours, filtered, and air dried at room temperature for 2 hours. The product was then slurried with $H_2O$ using a blender to ensure maximum mixing of the hydrophobic solid with water. The slurry was then transferred to a beaker and stirred overnight. The mixture was filtered and air dried for 4 hours.

The resulting filter cake was treated with tetraethylorthosilicate (TEOS) (5 g TEOS/g solid) for 72 hours. The pillared material was obtained by filtering this slurry and drying the solid in air. Calcination of the pillared material at 500 °C for about 4 hours in air eliminated octylamine and produced a silica-pillared titanozincate.

Table 1

Analysis of Products from N-Hexane Conversion

| Example No. | 1 | 2 | 3 | 4 |
|---|---|---|---|---|
| Catalyst | | | | |
| Temperature, °C | 538 | 538 | 538 | 538 |
| WHSV, $hr^{-1}$ | 1.4 | 1.4 | 1.4 | 1.35 |
| **Product Composition, wt%** | | | | |
| $H_2$ | 1.3 | 4.1 | 2.6 | 3.6 |
| $CH_4$ | 9.8 | 10.3 | 10.7 | 8.4 |
| $C_2H_6$ | 10.8 | 13.4 | 24.3 | 16.9 |
| $C_2^{=+}$ Non-Arom. | 41.5 | 16.3 | 12.9 | 13.0 |
| Benzene | 7.1 | 17.5 | 16.5 | 18.0 |
| Toluene | 15.4 | 22.1 | 16.9 | 23.8 |
| Xylenes | 9.7 | 10.8 | 10.4 | 11.5 |
| $C_9^+$ Aromatics | 4.2 | 5.5 | 5.6 | 4.7 |
| N-Hexane Conver. | 100 | 100 | 100 | 100 |
| Aromatics Select.* | 36 | 56 | 49 | 58 |
| Extinction Aromatics Selectivity** | 62 | 66 | 56 | 67 |

*Note: Since n-hexane conversion is 100% in all cases, aromatics selectivity is defined as (wt.% Benzene + wt% Toluene + wt% Xylene + wt% $C_9^+$ Aromatics).

**Note: Aromatics selectivity at extinction assumes that all of the $C_2^{=+}$ non-aromatics are completely converted to hydrogen, methane, ethane, and aromatics in the same proportion as that measured in the run. It is therefore defined here as follows:

$$\text{Extinction Aromatics Selectivity} = \frac{\text{Total Aromatics}}{100 - (C_2^{=+}\ \text{Non-Aromatics})}$$

Table 2

| Properties of Titanozincate Molecular Sieve | |
|---|---|
| Physical Properties | |
| Interlayer Opening<br>Surface Area, $m_2/g$ | 16.0 A<br>245 |
| Sorption (g/100g) | |
| $H_2O$<br>n-Hexane<br>Cyclohexane | 15.0<br>8.0<br>7.8 |
| Chemical Analysis | |
| Ti, wt%<br>$SiO_2$, wt%<br>Zn, wt%<br>Ash, wt%<br>K, wt% | 31.0<br>20<br>9.4<br>92.1<br>0.50 |

Example 5 (Comparative) $C_6/C_7$

2.5 g of HZSM-5 having a silica to alumina molar ratio of about 70 were impregnated with 0.35 g $Ga(NO_3)_3$. $9H_2O$ in sufficient deionized water to wet the sample, then dried at room temperature and then air calcined at 1000° F for two hours. The sample was used to process a $C_6/C_7$ naphtha, described in Table 7 at 1000° F and 0.5 LHSV. The product distribution of the reaction is shown in Table 8.

Example 6

(a) Solid State Preparation of Layered Titanogallate.

$Ga_2O_3$ (25.00 g, 0.133 mole), $TiO_2$ (31.95 g, 0.399 mole), and $Cs_2CO_3$ (43.43 g, .133 mole) were thoroughly mixed and ground to a homogeneous mixture. The mixture was fired at 910° C for 12 h after heating to 150° C for 2 h and 500° C for 4 h to decompose the reactants. Temperature ramps of 5° C/min were used. After firing, the product was cooled and ground. The x-ray powder diffraction pattern indicated that the product was a mixture of a layered phase and an additional material, possibly beta-$Ga_2O_3$. Analysis of the material gave the following composition: 34%Cs, 24.4% Ga, 20.3% Ti, 99.07% ash.

(b) Swelling with Octylammonium Ion

Concentrated HCl (66.65 g, 0.6912 mole) was diluted to 250 ml with $H_2O$. Octylamine (91.04 g, 0.7052 mole) was added slowly. Forty grams of the sample from (a) were added and the solution was heated to reflux with stirring for 48 h. The solution was filtered, washed with 1500 ml hot $H_2O$ and dried in air. The powder x-ray diffraction pattern from 2-20° 2 theta was obtained and indicated a d-spacing of 23.2 A in the solid.

This material (45.0 g) was stirred in 150 ml EtOH for 2 h, filtered, and air dried. The air dried sample was slurried in 300 ml $H_2O$ using a blender to ensure complete wetting of the hydrophobic solid. The slurry was transferred to a beaker and stirred overnight, then filtered and air dried.

(c) Treatment with Tetraethylorthosilicate (TEOS)

The solid from (b) was reslurried in 700 ml $H_2O$ for 2 h, filtered and air dried immediately prior to treatment with TEOS to ensure that the interlaminar water was present. The solid (36.94 g) was stirred in 185.0 g TEOS at 80°C for 24 h. The reaction was carried out under an $N_2$ environment to control the humidity. The reaction mixture was filtered and the solid dried in air to yield 39.66 g (7.4% weight uptake). The process was repeated; however, no weight gain was observed on the second treatment of this sample.

(d) Calcination to Produce a Molecular Sieve

The final product was obtained by calcining the sample at 500°C in $N_2$ for 1 h followed by 2 h in air. A 20.5% weight loss was observed during the final calcination. A powder x-ray diffraction pattern of the material showed a low angle at 3.9° 2 theta, indicating a basal spacing of 22.83 A and an interlayer separation of about 15.8 A assuming no degradation of the layer structure upon calcination. Chemical analysis of the material indicated the following composition: 9.47% Ga, 26.5% $SiO_2$, 21.3% Ti, 0.34% Cs, 98.00% ash. The porous solid had a surface area of 266$m^2$/g and absorbed 12.0% $H_2O$, 8.2% cyclohexane and 7.3% n-hexane.

Example 7

7.5 g HZSM-5, $SiO_2/Al_2O_3$ = 70.1, were physically mixed in a mortar and pestle with 2.5 g of the silica-pillared titanogallate material of Example 6. The physical mixture was pelleted and sized to 20/40 mesh and then used to process the $C_6/C_7$ naphtha described in Table 3 at 540°C (1000°F) and 0.5 LHSV. The product distributions are compared to those of Example 5 in Table 4.

Table 3

| Feedstock Properties | |
|---|---|
| Boiling Range | 180-250°F (82-121°C) |
| Density at 60°F (16°C) | 0.6914 |
| Hydrogen, wt.% | 15.55 |
| Sulfur, ppmw | 0.02 |
| Nitrogen, ppmw | 0.02 |
| Paraffins, wt.% | 81.3 |
| Naphthenes, wt.% | 13.3 |
| Aromatics, wt.% | 5.4 |
| $C_5$, wt.% | 2.7 |
| $C_6$, wt.% | 49.8 |
| $C_7$, wt.% | 47.2 |
| $C_8$, wt.% | 0.3 |

Table 4

| Product Distributions | | |
|---|---|---|
| Wt.% of Products | Example 5 | Example 7 |
| $H_2$ | 6.6 | 3.9 |
| $CH_4$ | 4.5 | 4.4 |
| $C_2H_6$ | 12.3 | 9.5 |
| $C_2^{=+}$ | 21.9 | 40.2 |
| Benzene | 12.4 | 5.5 |
| Toluene | 21.6 | 14.7 |
| Xylenes | 11.8 | 10.2 |
| $C_9^+$ | 8.7 | 11.6 |
| Aromatic Selectivity | 69 | 70 |

Example 8

A $TiO_2$ catalyst was impregnated with one weight percent zinc by impregnating 10 g $TiO_2$ with 0.3 g of $Zn(NO_3)_2$ in deionized water. The sample was evaporated to dryness and then air calcined at 538° C for two hours.

A sample of ZnZSM-5 from Example 2 (one weight percent Zn) and a sample of the mixture of silica-pillared titanozincate (25 weight percent) and HZSM-5 (75 weight percent) containing one weight percent zinc from Example 4, and the Zn-impregnated $TiO_2$ catalyst were exposed to flowing hydrogen at 593° C (1100° F) for up to about 32 hours (atmospheric pressure, 100% hydrogen atmosphere, 10.5 cm/second linear flow velocity of gas. The Zn/$TiO_2$ catalyst lost a significant fraction of its metal in less two hours. After four hours no zinc was detected on the support. In contrast, the silica-pillared titanozincate/ZSM-5 lost its zinc more slowly and appeared to lose a maximum of 75% of the original zinc. Very little additional zinc was lost after 16 hours under the reducing conditions used in these experiments.

The Figure depicts the Zn loss from these three various zinc-containing catalysts over a 32 hour period.

Example 9

The silica-pillared titanozincate of Example 4 and the silica-pillared titanogallate of Example 6 were tested for their ability to retain Zn or Ga under the reducing conditions employed in Example 8. The results for the silica-pillared titanozincate given below in Table 5 show that Zn is eluted slower than Zn from $TiO_2$ and faster than Zn from the silica-pillared titanozincate/ZSM-5 mixture or ZnZSM-5. The results for the silica-pillared titanogallate set out in Table 6 below show that substantially no gallium was eluted, even after 32 hours. Accordingly, silica-pillared titanogallates appear to be especially well-suited to use under reducing conditions where it is desirable to avoid elution of the metal function.

Table 5

| Silica-Pillared Titanozincate-Elution Studies 594° C, 200 cc/min $H_2$ at atmospheric pressure | | |
|---|---|---|
| Hours in $H_2$ | Wt.% Zn | % Zn Remaining |
| 0 | 6.2, 5.9 | 100 |
| 1 | 4.5 | 72 |
| 2 | 3.5 | 56 |
| 4 | 0.97 | 15 |
| 8 | 0.33 | 5.3 |
| 16 | 0.25 | 4.0 |
| 32 | 0.18 | 3.0 |

Table 6

| Silica-Pillared Gallium Titanate Elution Studies 594° C, 200 cc/min $H_2$ at atmospheric pressure | |
|---|---|
| Hours in $H_2$ | Wt.% Ga |
| 0 | 19.6 |
| 2 | 19.5 |
| 4 | 21.2 |
| 8 | 21.0 |
| 16 | 20.8 |
| 32 | 20.3 |

Example 10

In this example, the layered starting material was a titanate having the empirical formula $Cs_{0.7}Ti_{1.82}O_4$. This material contains vacancies at certain titanium sites in the layers and so can be described by the general formula $Cs_{4y}(\square_y Ti_{2-y})O_4$ wherein □ is a vacancy site and y is 0.18.

The layered vacancy titanate was prepared by the high temperature solid state reaction of $Cs_2CO_3$ and $TiO_2$ in the stoichiometry of 1:5.2. The $Cs_2CO_3$ employed was ground to fine powder (less than 100 mesh) dried and stored in a vacuum oven at 180° C. The $TiO_2$ employed was used as received. The solids (50g$Cs_2CO_3$ and 63.93g $TiO_2$)were ground to an homogenous mixture which was fired at 650° C for 10 hours and, after regrinding, was then fired at 950° C for a further 10 hours. The resultant product was then ground.

30g of the titanate product was then swollen by replacing with octylamine/HCl (mole ratio 1 titanate: 5 octylamine:4.9HCL) for 14 hours. After washing with 1000 ml of water, the product was dried in air overnight.

25g of the swelled titanate was stirred in 300 ml ethanol, filtered and air dried. The dried solid was then slurried in 500 ml water for 24 hours, pillared and air dried overnight. The resultant solid (16.4g) was stirred with 100g of TEOS for 24 hours and the mixture was filtered and air dried to yield 18.5 g of solid product. The required porous molecular sieve was obtained by calcining the product in air at 500°C for 4 hours. This sieve is then mixed with H ZSM-5 and pelleted, with the resulting material used to process $C_6/C_7$ naptha according to the same procedure described in Example 7.

## Claims

1. A catalyst composition which comprises a) a porous crystalline silicate having a Constraint Index of at least about 1 and b) a pillared metal oxide material comprising a layered metal oxide and pillars of a chalcogenide of at least one element selected from Groups IB, IIB, IIIA, IIIB, IVA, IVB, VA, VB, VIA, VIIA and VIIIA of the Periodic Table of the Elements separating the layers of the metal oxide, wherein each layer of the metal oxide has the general formula

$[M_x\square_yZ_{2-(x+y)}O_4]^{q-}$

wherein M is at least one metal of valence n wherein n is an integer between 0 and 7, $\square$ represents a vacancy site, Z is a tetravalent metal, and wherein

$q = 4y-x(n-4)$

$0 < x+y < 2$

2. The composition of claim 1 wherein said crystalline silicate has a Constraint Index of 1 to 12.

3. The composition of claim 1 wherein said porous crystalline silicate material has the structure selected from the group consisting of ZSM-5, ZSM-11, ZSM-12, ZSM-22, ZSM-23, ZSM-35, ZSM-48, ZSM-50, ZSM-57, and ZSM-58.

4. The composition of any preceding claim wherein Z is titanium.

5. The composition of claim 4 wherein y is zero.

6. The composition of claim 1 wherein q is from 0.6-0.9.

7. The composition of any preceding claim wherein M is selected from the group consisting of Ga and Zn.

8. The composition of any preceding claim wherein the pillars comprise a polymeric oxide.

9. The composition of any preceding claim wherein the pillars comprise polymeric silica.

10. A process for producing aromatic hydrocarbons which comprises contacting a feed containing non-aromatic $C_2$ to $C_{12}$ hydrocarbons with the composition of any preceding claim at a pressure of 100-7000 kPa (atmospheric to 1000 psig), a weight hourly space velocity of about 0.05 to 300 and a temperature of about 204 to 675°C.

Neu eingereicht / Newly filed
Nouvellement déposé

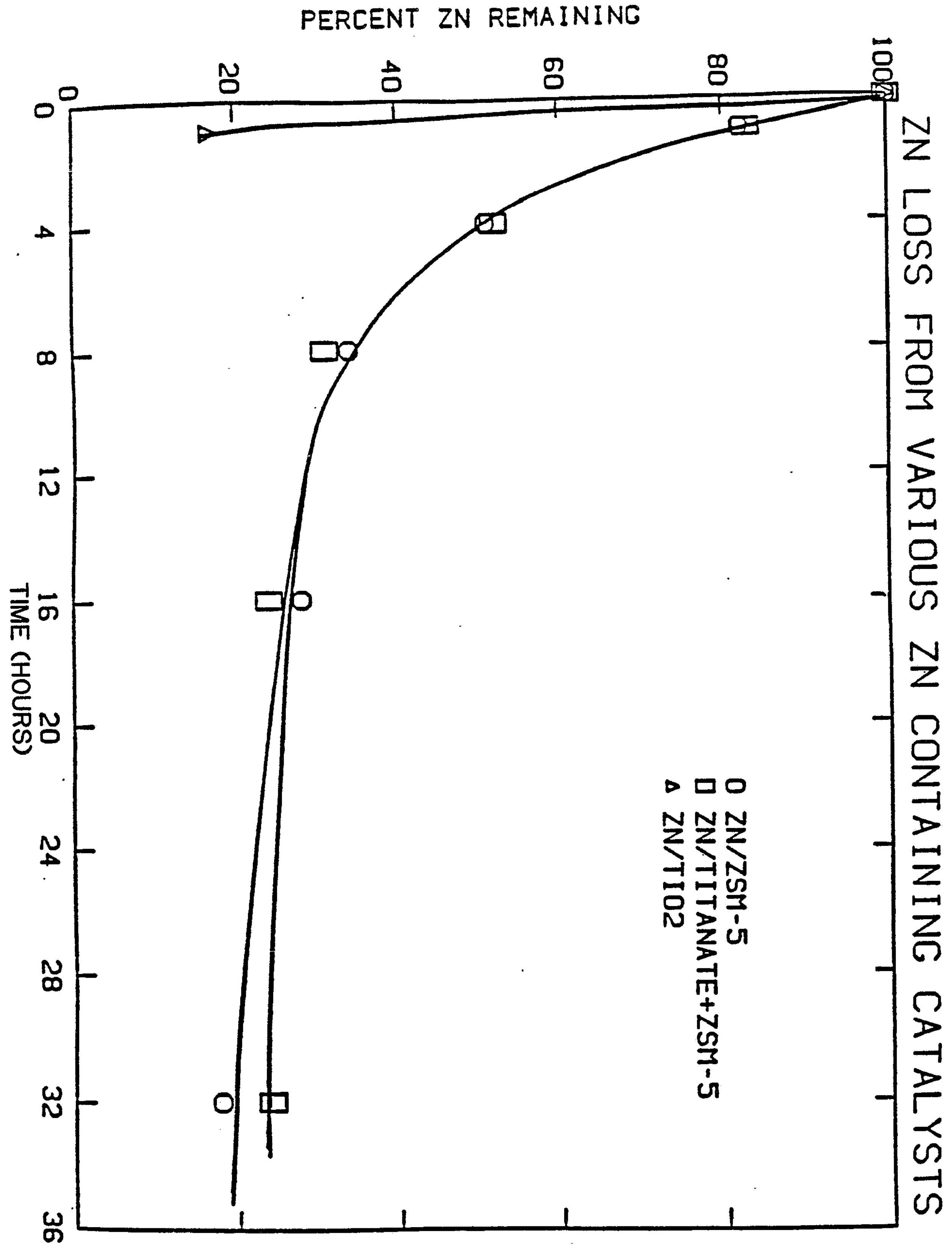

European Patent Office

# EUROPEAN SEARCH REPORT

Application Number

EP 88 30 6610

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | WO-A-8 800 092 (MOBIL OIL)<br>--- | | B 01 J 29/00 //<br>C 07 C 2/12<br>C 07 C 15/00 |
| A | EP-A-0 225 686 (MOBIL OIL)<br>--- | | |
| A | EP-A-0 110 628 (B.P.)<br>--- | | |
| A | US-A-4 742 033 (J.R. HARRIS)<br>--- | | |
| A | FR-A-2 581 567 (C.F.R.)<br>----- | | |
| | | | **TECHNICAL FIELDS SEARCHED (Int. Cl.4)** |
| | | | B 01 J 29/00<br>C 07 C 2/00<br>C 07 C 15/00 |

The present search report has been drawn up for all claims

| Place of search | Date of completion of the search | Examiner |
|---|---|---|
| THE HAGUE | 23-03-1989 | DEVISME F.R. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document